# EUROPEAN PATENT APPLICATION

(11) **EP 3 578 218 A1**
(43) Date of publication of application: **11.12.2019**
(21) Application number: 18176856.5
(22) Date of filing: 08.06.2018
(51) Int. Cl.: A61M 15/00

(54) **INHALER**

(71) Applicant: Presspart Manufacturing S.A., 43720 L'Arboc del Penedes, Tarragona (ES)
(72) Inventor: TORRES MUNIESA, Victor, 43720 L'Arboç, Tarragona (ES); HERRERA MARTÍNEZ, Paloma, 43720 L'Arboç, Tarragona (ES); BUSTO SOTIL, Santiago, 43720 L'Arboç, Tarragona (ES); VALENCIA PELLISA, David, 43720 L'Arboç, Tarragona (ES)
(74) Representative: Uexküll & Stolberg

(57) **Abstract**

An inhaler (1) has a hollow inhaler body (2) for retaining an aerosol container with a dispensing valve at a valve end of the container. The inhaler comprises a first open end (3) sized and arranged to receive the aerosol container with a dispensing valve and a second open end (4) through which a dose of medicament is dispensed as an aerosol. The second open end (3) is sized and arranged to be coupled to the mouth or nasal cavities of a patient. The inhaler (1) further comprises a nozzle block (5) which extends from a bottom portion (6) of the inhaler body (2) towards the first open end (3) and against which the dispensing valve of the aerosol container is depressed to release a dose of a medicament. The nozzle block (5) has a fluid flow path (7) extending therethrough such that the dose of medicament being released from the valve is passed to the nozzle block (5) and exits the nozzle block (5) as an aerosol in a direction towards the second open end (4) of the inhaler body (2).

The nozzle block (5) is formed as a part separate from the inhaler body (2). The bottom portion (6) of the inhaler body (2) comprises a receiving portion (11) for receiving the nozzle block (5). The nozzle block (5) is adapted to be inserted through the first open end (3) of the inhaler body (2) into the receiving portion (11) of the bottom portion (6).

## Description

### TECHNICAL FIELD

The present invention relates to an inhaler having a hollow inhaler body for retaining an aerosol container with a dispensing valve at a valve end of the container. The inhaler body comprises a first open end sized and arranged to receive the aerosol container with a dispensing valve and a second open end through which a dose of medicament is dispensed as an aerosol. The second open end is sized and arranged to be coupled to the mouth or nasal cavities of a patient. The inhaler body further comprises a nozzle block which extends from a bottom portion of the inhaler body towards the first open end and against which the dispensing valve of the aerosol container is depressed to release a dose of a medicament. The nozzle block has a fluid flow path extending therethrough such that the dose of medicament being released from the valve is passed to the nozzle block and exits the nozzle block as an aerosol in a direction towards the second open end of the inhaler body.

### BACKGROUND OF THE INVENTION

Inhalers, in particular metered dose inhalers (MDIs), are medication delivery devices which deliver a pharmaceutical formulation including one or more pharmaceutical active compounds to a human or another mammalian patient. Typically, the pharmaceutical formulation is delivered by the MDIs in unit doses in the form of an aerosol. Each actuation of the MDIs delivers one unit dose. The unit dose is expelled by the MDIs and is taken into the body of the patient on inhalation, via the nose or mouth. The pharmaceutical formulation is delivered to or via the respiratory tract, notably to the lungs, of the patient on inhalation. Metered dose inhalers are typically used for the treatment of respiratory infections and disorders including respiratory tract infections, obstructive lung disease, inflammatory lung disease and chronic obstructive pulmonary disease. Asthma treatment is a particularly common use of MDIs

ES 1069857 U discloses such a metered dose inhaler having an angular body with a notably cylindrical vertical part and a lower part. The lower part protrudes forward, constituting an outlet opening forming an angle of greater than 90° with the vertical part. An outlet structure is positioned on the lower base of the vertical part and comprises several holes in order to direct aerosol from a container being received in the vertical part to the outlet opening.

Typically, inhalers are manufactured according to customer specifications. After delivery to the customer the inhalers are then equipped with an aerosol container and brought to the market. Specifications of different customers often only vary with regard to the design of the nozzle block. This is because the design of the nozzle block significantly influences the spray pattern of the aerosol exiting the nozzle towards the second open end of the inhaler body. Moreover, the nozzle block design depends amongst others on the formulation of the medicament to be used in combination with the inhaler. However, the whole inhaler has to be designed and manufactured individually in order to address the needs of every customer which is rather time consuming and expensive.

Moreover, inhalers are preferably manufactured under use of the injection molding technique by which melted plastic particles are injected into moulds where they solidify and take the appearance of an inhaler body. As the geometry of an inhaler body comprises several openings and undercuts its geometry is quite complex. Accordingly, the moulds comprise several parts which are movable relative to one another in order to allow removement of the inhaler body from the mould. Consequently tooling, in particular the moulds used for the injection molding, are very expensive.

It is an object of the present invention to provide a cost effective inhaler which allows a simplified production and addresses individual customer needs.

### SUMMARY OF THE INVENTION

This object is achieved by an inhaler comprising the features of claim 1, preferred embodiments are set out in the dependent claims.

According to the present invention the nozzle block is formed as a part separate from the inhaler body. The bottom portion of the inhaler body comprises a receiving portion for receiving the nozzle block. The nozzle block is adapted to be inserted through the first open end of the inhaler body into the receiving portion of the bottom portion. The bottom portion and the inhaler body are integrally formed, i.e. as one piece. Preferably, the inhaler body together with its bottom portion is formed via injection molding.

As the nozzle block is formed as a separate part from the inhaler body, the nozzle block can be individually designed according to customer needs, whereas the inhaler body stays the same. Additionally, the nozzle block may be designed as a part being compatible with a range of inhaler bodies having different sizes and/or different shapes. Moreover, the design of the nozzle block can be optimized with regard to the manufacturing process, in particular with regard to the injection molding of the nozzle block, without having to consider producibility of the inhaler body together with the nozzle block in one single mould. This allows cheaper tooling and decreases the production costs. Preferably, the outer shape of the nozzle block, at least in the area which is received by the receiving portion of the inhaler body, stays unchanged in order to ensure a proper fit of the nozzle block in the receiving portion. Optionally, the design changes of the nozzle block for addressing customer needs relate to the fluid flow path extending therethrough.

Preferably, the inhaler according to the present invention may be used as a metered dose inhaler with or without an additional dose counter which may be a mechanical and/or electronic dose counter. Optionally, a mechanical dose counter is located vertically above the nozzle block. Alternatively or additionally an electronic dose counter may be located between a rear side of the inhaler body and the aerosol container or a front side of the inhaler body and the aerosol container. Preferably, the mechanical dose counter is mounted on the bottom portion of the inhaler body and is inserted into the inhaler body through the first open end.

In an embodiment of the present invention the nozzle block is connected to the bottom portion of the inhaler body via a form fitting connection. Preferably the nozzle block is inserted into the inhaler body through its first open end and into the receiving portion from above. The form fitting connection may be configured to withstand the forces which occur during operation of the inhaler, in particular during depression and release of the dispensing valve. The form fitting connection allows an easy assembling of the nozzle block and the inhaler body.

In another embodiment of the present invention the nozzle block has an at least partially cylindrical shape, wherein the nozzle block comprises a radially outward extending lower edge which is adapted to engage with a slot in the receiving portion of the bottom portion to form the form fitting connecting. Preferably, the lower edge is part of a snap in tongue of the nozzle block. The snap in tongue is configured such that the lower edge snaps into the slot of the receiving portion upon insertion of the nozzle block into the receiving portion. Upon engagement of the lower edge and the slot axial movement of the nozzle block towards the first open end is omitted.

Preferably, the nozzle block has an upper portion, and a lower portion positioned adjacent to one another. Optionally, the upper portion receives the dispensing valve of the aerosol container, whereas the aerosol exits the nozzle block in the area of the lower portion towards the second open end of the inhaler body.

In an embodiment the nozzle block has a supporting leg for vertically supporting the nozzle block in the receiving portion, wherein the supporting leg comprises a T-shaped cross sectional area having a stem and two opposing arms, wherein the stem forms a rib which extends from the upper portion along the lower portion and, wherein the rib is adapted to engage with a vertically extending groove in the receiving portion. The T-shapes cross sectional area extends lateral to an axis defined by the nozzle block extending from the bottom portion towards the first open end of the inhaler body. Upon engagement of the rib and the vertically extending groove in the receiving portion the orientation of the nozzle block around the axis defined by the nozzle block extending from the bottom portion towards the first open end of the inhaler body is set. This ensures that a dose of a medicament being released from the valve of the aerosol container exits the nozzle block as an aerosol in a direction towards the second open end of the inhaler body. The rib of the nozzle block and the groove in the receiving portion are engaged upon insertion of the nozzle block into the receiving portion.

In a preferred embodiment the upper portion comprises opposing recesses, which each have a plane base surface, wherein the base surfaces are arranged parallel to one another. Preferably the base surfaces are arranged parallel to the axis defined by the nozzle block extending from the bottom portion towards the first open end of the inhaler body.

In another embodiment the receiving portion of the bottom portion has an essentially cylindrical shape and comprises opposing protrusions which protrude radially inward. Preferably, the inner shape of the receiving portion is formed corresponding to the outer shape of the nozzle block.

Preferably, the opposing recesses of the nozzle block are configured to engage with the opposing protrusions of the receiving portion upon insertion of the nozzle block into the receiving portion. Optionally, the protrusions form a stop for the recesses and thus keep the nozzle block in an upright position in the receiving portion such that the nozzle block points towards the first open end of the inhaler body. As the engagement of the recesses and the protrusions keeps the nozzle block in the upright position its lower edge is kept in engagement with the slot of the receiving portion. Thus, the engagement of the recesses and the protrusions prevents movement of the nozzle block along the axis defined by the nozzle block extending from the bottom portion towards the first open end of the inhaler body.

According to another embodiment the fluid flow path of the nozzle block is formed by a sump configured to receive the dispensing valve of the aerosol container, a nozzle configured to aerosolize the dose of medicament being released from the dispensing valve and a channel extending from the sump to the nozzle. In order to ensure a proper functioning of the nozzle block and that the molded nozzle block is dimensionally within the specification, it is, among other dimensions, required to measure the diameter at the transition of the channel to the nozzle (orifice diameter) as well as the length of the channel (jet length).

In an embodiment of the present invention the nozzle block is formed of transparent material. This allows visual inspection of the nozzle block after fabrication. In particular, this allows the measurement of the nozzle block by the use of vision systems which don't require a specific handling or physical change (e.g. cutting, adding liquids for increasing contrast etc.). This enables a cheap, fast and 100% inline inspection check for nozzle blocks and thus total batch traceability. Vision systems typically allow to capture a digital image of a part which is to be measured. The measurements are then taken from the image manually or automatically in a subsequent step. Preferably the vision system comprises a camera or any other device which is capable of taking an image of the part to be measured.

Preferably, the lower portion of the nozzle block comprises opposing plane side surfaces which are arranged parallel to one another. The plane side surfaces allow recording of a non-distorted image of the interior of the nozzle block as the rays, in particular light rays, enter and exit the nozzle block preferably at a right angle with regard to the plane side surfaces such that the rays are not deflected. This enables true measurements of the interior of the nozzle block.

In another preferred embodiment the side surfaces of the lower portion are arranged parallel to a first axis defined by the channel connecting the sump and the nozzle of the nozzle block and are arranged parallel to a second axis defined by the nozzle block extending from the bottom portion towards the first open end of the inhaler body. Such an arrangement of the side surfaces allows direct measuring of the jet length as well as the orifice in between the channel and the nozzle.

In another embodiment the inhaler comprises an insert configured to be inserted into the inhaler body through its first open end, wherein the insert has ribs which extend from the first open end towards the bottom portion of the inhaler body and which are adapted to abut the aerosol container upon insertion into the inhaler body. Preferably, the insert is available in different sizes which differ in the size of the ribs in between the inhaler body and the aerosol container and thus in a radial direction, namely in a direction lateral to the second axis. The insert allows the adaption of the inhaler body to receive aerosol containers of different sizes, in particular of different diameters. Thus, by use of inserts having different sizes one single inhaler body may be adapted to accommodate aerosol containers of different sizes, thereby reducing costs of tooling and production costs.

Preferably, the inhaler body has a bottom side, and comprises corrugations thereon. The area at the bottom side of the inhaler body is often used by patients to hold the inhaler during inhalation. As the corrugations allow secure gripping of the inhaler during inhalation an accidently dropping of the inhaler is avoided.

The invention will now be described in connection with one exemplary embodiment shown in the figures in which:
- Figure 1: shows a perspective view of an inhaler,
- Figure 2: shows a sectional side view of the inhaler,
- Figure 3: shows a perspective view of a nozzle block,
- Figure 4: shows a front view of the nozzle block of Figure 3,
- Figure 5: shows a perspective rear view of the nozzle block of Figure 3,
- Figure 6A: shows a sectional top view along the line A-A in Figure 2,
- Figure 6B: shows a detail Y of Figure 6A
- Figure 7A: shows a sectional top view along the line B-B in Figure 2 and
- Figure 7B: shows a detail Z of Figure 7A.

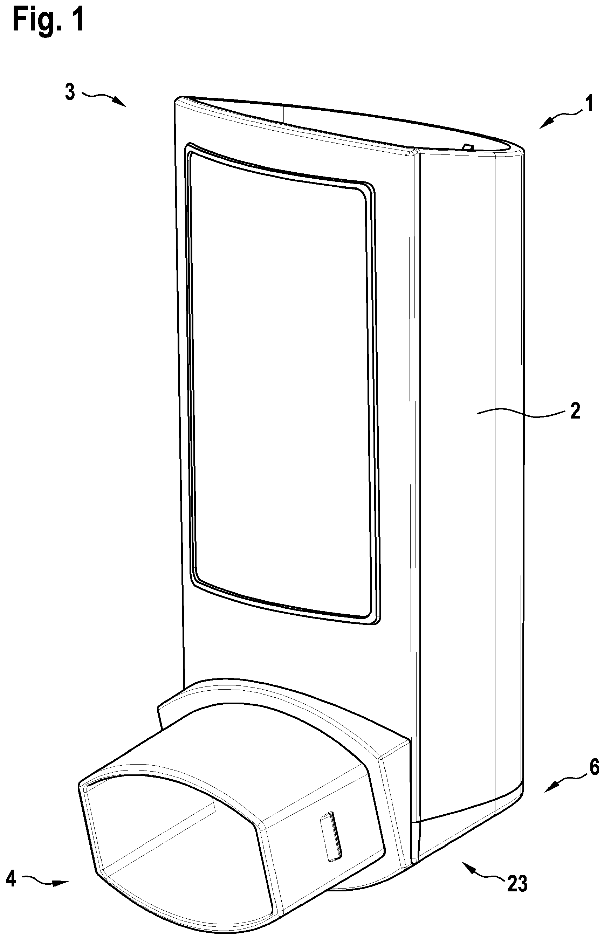

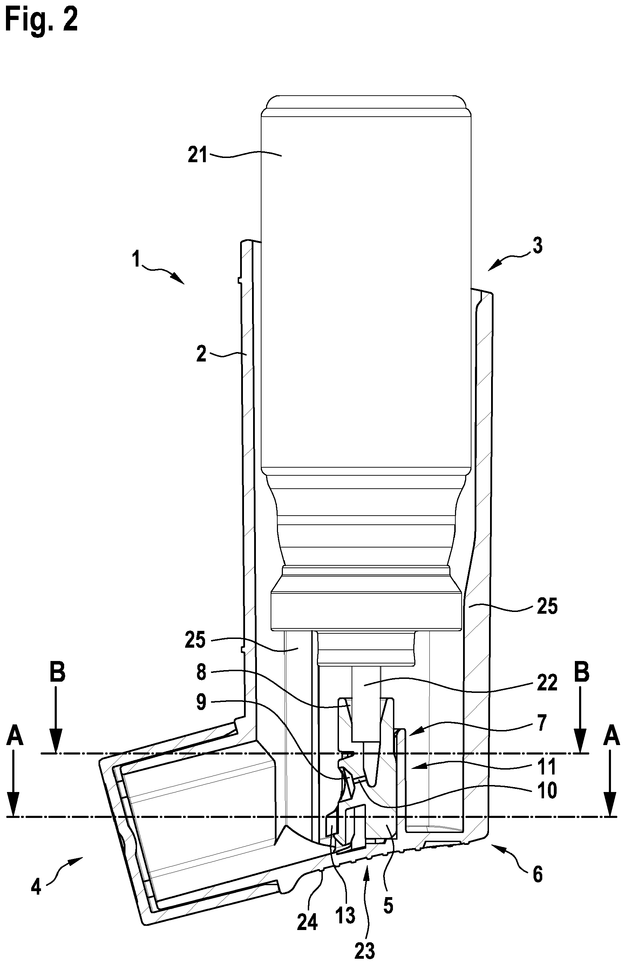

Figures 1 and 2 show an inhaler 1 with a hollow inhaler body 2 for retaining an aerosol container 21 with a dispensing valve 22 at a valve end of the container. The inhaler 1 comprises a first open end 3 sized and arranged to receive the aerosol container 21 with a dispensing valve 22 and a second open end 4 through which a dose of a medicament is dispensed as an aerosol. The second open end 4 is sized and arranged to be coupled to the mouth or nasal cavities of a patient (not shown). The inhaler body 2 has a bottom side 23 and comprises corrugations 24 thereon. The inhaler body 2 forms an angular hollow tubular body extending from the first open end 3 towards the second open end 4.

The inhaler body 2 further comprises ribs 25 which extend from the first open end 3 towards a bottom portion 6 of the inhaler body 2 and which are adapted to abut the aerosol container 21 upon its insertion into the inhaler body 2. Preferably, the ribs 25 are arranged on an insert (not shown) configured to be inserted into the inhaler body 2 through its first open end 3. Optionally, the insert may be exchangeable such that multiple inserts with different sizes of the ribs 25 may alternatively be inserted into the inhaler body 2 in order to accommodate aerosol containers 21 of different sizes.

The inhaler body 2 further comprises a nozzle block 5 which extends from the bottom portion 6 of the inhaler body 2 towards the first open end 3 and against which the dispensing valve 22 of the aerosol container 21 is depressed to release a dose of medicament. The nozzle block 5 has a fluid flow path 7 extending therethrough such that the dose of medicament being released from the valve 22 is passed to the nozzle block 5 and exits the nozzle block 5 as an aerosol in a direction towards the second open end 4 of the inhaler body 2.

The fluid flow path 7 of the nozzle block 5 is formed by a sump 8 configured to receive the dispensing valve 22 of the aerosol container, a nozzle 9 configured to aerosolize the medicament being released from the dispensing valve 22 and a channel 10 extending from the sump 8 to the nozzle 9.

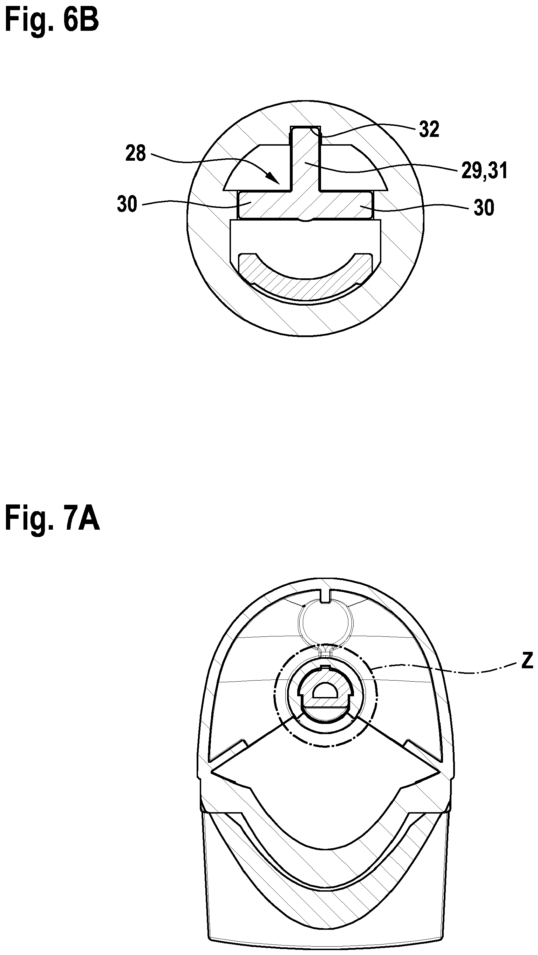

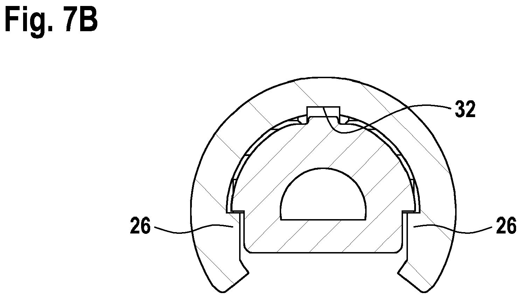

The nozzle block 5 is formed as a part separate from the inhaler body 2 and is adapted to be inserted through the first open end 3 of the inhaler body 2 into a receiving portion 11 at the bottom portion 6 of the inhaler body 2. The nozzle block 5 is connected to the bottom portion 6 of the inhaler body 2 via a form fitting connection. The receiving portion 11 of the bottom portion 6 has an essentially cylindrical shape and comprises opposing protrusions 26 (Figure 7B) which protrude radially inward.

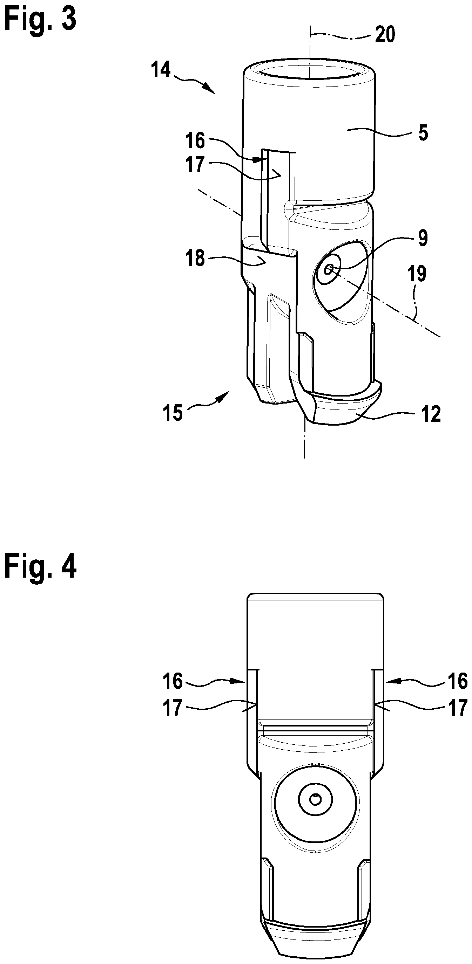

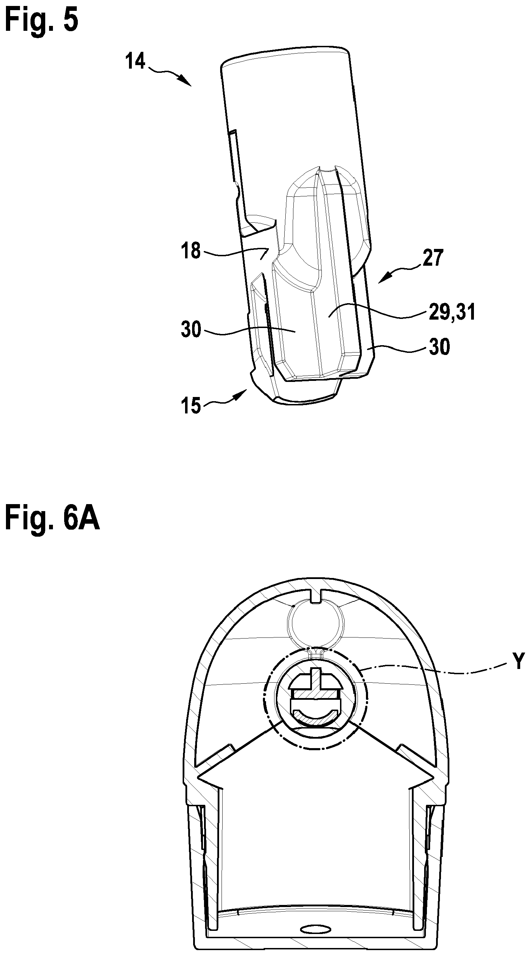

Figures 3 to 7A show the nozzle block 5 and its position in the receiving portion 11 in detail.

The nozzle block 5 has an at least partially cylindrical shape. It comprises a radially outward extending lower edge 12 which is adapted to engage with a slot 13 (Figure 2) in the receiving portion 11 of the bottom portion 6 to form the form fitting connecting between the nozzle block 5 and the inhaler body 2.

The nozzle block 5 has an upper portion 14, and a lower portion 15 positioned adjacent to one another. The nozzle block 5 comprises at its upper portion 14 opposing recesses 16, which each have a plane base surface 17, wherein the base surfaces 17 are arranged parallel to one another.

The opposing recesses 16 of the nozzle block 5 are configured to engage with the opposing protrusions 26 of the receiving portion 11 upon insertion of the nozzle block 5 into the receiving portion 11.

The nozzle block 5 has a supporting leg 27 for vertically supporting the nozzle block 5 in the receiving portion 11. The supporting leg 27 comprises a T-shaped cross sectional area 28 having a stem 29 and two opposing arms 30. The stem 29 forms a rib 31 which extends from the upper portion 14 along the lower portion 15. The rib 31 is adapted to engage with a vertically extending groove 32 in the receiving portion 11.

The nozzle block 5 further comprises at its lower portion 15 opposing plane side surfaces 18 which are arranged parallel to one another. The side surfaces 18 of the lower portion 15 are arranged parallel to a fist axis 19 defined by the channel 10 connecting the sump 8 and the nozzle 9 of the nozzle block 5 and are arranged parallel to a second axis 20 defined by the nozzle block 5 extending from the bottom portion 6 towards the first open end 3 of the inhaler body 5. The nozzle block 5 may be formed of transparent material.

### Reference numerals

- 1: inhaler
- 2: inhaler body
- 3: first open end (inhaler body)
- 4: second open end (inhaler body)
- 5: nozzle block
- 6: bottom portion (inhaler body)
- 7: fluid flow path (nozzle block)
- 8: sump (nozzle block)
- 9: nozzle (nozzle block)
- 10: channel (nozzle block)
- 11: receiving portion (inhaler body)
- 12: lower edge (nozzle block)
- 13: slot (receiving portion)
- 14: upper portion (nozzle block)
- 15: lower portion (nozzle block)
- 16: recesses (nozzle block)
- 17: base surface (recesses)
- 18: side surface (nozzle block)
- 19: first axis (nozzle block)
- 20: second axis (nozzle block)
- 21: aerosol container
- 22: dispensing valve
- 23: bottom side (inhaler body)
- 24: corrugations (inhaler body)
- 25: ribs
- 26: opposing protrusions (receiving portion)
- 27: supporting leg (nozzle block)
- 28: cross sectional area (supporting leg)
- 29: stem (cross sectional area)
- 30: arms (cross sectional area)
- 31: rib (cross sectional area)
- 32: groove (receiving portion)

## Claims

1. An inhaler having a hollow inhaler body (2) for retaining an aerosol container (21) with a dispensing valve (22) at a valve end of the container (21), the inhaler body (2) comprising:
- a first open end (3) sized and arranged to receive the aerosol container (21) with a dispensing valve (22);
- a second open end (4) through which a dose of medicament is dispensed as an aerosol, the second open end (4) being sized and arranged to be coupled to the mouth or nasal cavities of a patient;
- a nozzle block (5) which extends from a bottom portion (6) of the inhaler body (2) towards the first open end (3) and against which the dispensing valve (22) of the aerosol container (21) is depressed to release a dose of a medicament, the nozzle block (5) having a fluid flow path (7) extending therethrough such that the dose of medicament being released from the valve (22) is passed to the nozzle block (5) and exits the nozzle block (5) as an aerosol in a direction towards the second open end (4) of the inhaler body (2);
**characterized in**
**that** the nozzle block (5) is formed as a part separate from the inhaler body (2),
**that** the bottom portion (6) of the inhaler body (2) comprises a receiving portion (11) for receiving the nozzle block (5) and
**that** the nozzle block (5) is adapted to be inserted through the first open end (3) of the inhaler body (2) into the receiving portion (11) of the bottom portion (6).

2. Inhaler according to claim 1, **characterized in that** the nozzle block (5) is connected to the bottom portion (6) of the inhaler body (2) via a form fitting connection.

3. Inhaler according to claim 2, **characterized in that** the nozzle block (5) has an at least partially cylindrical shape, wherein the nozzle block (5) comprises a radially outward extending lower edge (12) which is adapted to engage with a slot (13) in the receiving portion (11) of the bottom portion (6) to form the form fitting connecting.

4. Inhaler according to any of the preceding claims, **characterized in that** the nozzle block (5) has an upper portion (14), and a lower portion (15) positioned adjacent to one another.

5. Inhaler according to claim 4, **characterized in that** the nozzle block (2) has a supporting leg (27) for vertically supporting the nozzle block (5) in the receiving portion (11), wherein the supporting leg (27) comprises a T-shaped cross sectional area (28) having a stem (29) and two opposing arms (30), wherein the stem (29) forms a rib (31) which extends from the upper portion (14) along the lower portion (15) and, wherein the rib (31) is adapted to engage with a vertically extending groove (32) in the receiving portion (11) .

6. Inhaler according to claims 4, **characterized in that** the upper portion (14) comprises opposing recesses (16), which each have a plane base surface (17), wherein the base surfaces (17) are arranged parallel to one another.

7. Inhaler according to any of the preceding claims, **characterized in that** the receiving portion (11) of the bottom portion (6) has an essentially cylindrical shape and comprises opposing protrusions (26) which protrude radially inward.

8. Inhaler according to claims 6 and 7, **characterized in that** the opposing recesses (16) of the nozzle block (5) are configured to engage with the opposing protrusions (26) of the receiving portion (11) upon insertion of the nozzle block (5) into the receiving portion (11).

9. Inhaler according to any of the preceding claims, **characterized in that** the fluid flow path (7) of the nozzle block (5) is formed by a sump (8) configured to receive the dispensing valve (22) of the aerosol container (21), a nozzle (9) configured to aerosolize the dose of medicament being released from the dispensing valve (22) and a channel (10) extending from the sump (8) to the nozzle (9).

10. Inhaler according to any of the preceding claims, **characterized in that** the nozzle block (5) is formed of transparent material.

11. Inhaler according to claim 4, **characterized in that** the lower portion (15) of the nozzle block (5) comprises opposing plane side surfaces (18) which are arranged parallel to one another.

12. Inhaler according to claims 8 and 9, **characterized in that** the side surfaces (18) of the lower portion (15) are arranged parallel to a fist axis (19) defined by the channel (10) connecting the sump (8) and the nozzle (9) of the nozzle block (5) and are arranged parallel to a second axis (20) defined by the nozzle block (5) extending from the bottom portion (6) towards the first open end (3) of the inhaler body (2).

13. Inhaler according to any of the preceding claims, **characterized in that** the inhaler (1) comprises an insert configured to be inserted into the inhaler body (2) through its first open end (3), wherein the insert has ribs (25) which extend from the first open end (3) towards the bottom portion (6) of the inhaler body (2) and which are adapted to abut the aerosol container (21) upon insertion into the inhaler body (2).

14. Inhaler according to any of the preceding claims, **characterized in that** the inhaler body (2) has a bottom side (23), and comprises corrugations (24) thereon.
